# EUROPEAN PATENT SPECIFICATION

(11) **EP 3 861 937 B1**
(45) Date of publication and mention of the grant of the patent: **11.10.2023**
(21) Application number: 21150960.9
(22) Date of filing: 27.03.2019
(51) Int. Cl.: A61B 8/12, A61B 1/018, A61B 1/267, A61B 8/00, A61B 10/02, A61B 34/00, A61B 17/00, A61B 90/00, A61B 34/20

(54) **ELECTROMAGNETIC NAVIGATION BRONCHOSCOPY USING ULTRASOUND**
BRONCHOSKOPIE MIT ELEKTROMAGNETISCHER NAVIGATION UNTER VERWENDUNG VON ULTRASCHALL
BRONCHOSCOPIE PAR NAVIGATION ÉLECTROMAGNÉTIQUE À L'AIDE D'ULTRASONS

(30) Priority: 28.03.2018 US 201862648992 P; 12.03.2019 US 201916351075
(43) Date of publication of application: 11.08.2021
(62) Divisional of application: 19165475.5
(73) Proprietor: Covidien LP, Mansfield, MA 02048 (US)
(72) Inventor: KOPEL, Evgeni, 46120 Herzliya (IL); KLEIN, Eyal, 6955306 Tel Aviv (IL); WEINGARTEN, Oren P., 46120 Herzliya (IL); GREENBURG, Benjamin, 45217 Hod Hasharon (IL)
(74) Representative: Maschio & Soames IP Ltd

(56) References cited:
- US-A1- 2016 000 414
- US-A1- 2017 319 772
- US-A1- 2018 140 359

## Description

### BACKGROUND

### Technical Field

Example aspects described herein relate generally to integrating ultrasound with electromagnetic navigation bronchoscopy, and, more particularly, to systems, methods, and computer-readable media for facilitating electromagnetic navigation bronchoscopy using ultrasound to locate and navigate to a target tissue.

### Description of Related Art

A bronchoscope is commonly used to inspect an airway of a patient. Typically, the bronchoscope is inserted into the patient's airway through the patient's nose or mouth or another opening, and can extend into the lungs of the patient. The bronchoscope typically includes an elongated flexible tube having an illumination assembly for illuminating the region distal to the bronchoscope's tip, an imaging assembly for providing a video image from the bronchoscope's tip, and a working channel through which an instrument, such as a diagnostic instrument (for example, a biopsy tool), a therapeutic instrument, and/or another type of tool, can be inserted.

Electromagnetic navigation (EMN) systems and methods have been developed that utilize a three-dimensional model (or an airway tree) of the airway, which is generated from a series of computed tomography (CT) images generated during a planning stage. One such system has been developed as part of Medtronic Inc.'s ILOGIC^{®} ELECTROMAGNETIC NAVIGATION BRONCHOSCOPY^{®} (ENB^{™}) system. The details of such a system are described in U.S. Patent No. 7,233,820, entitled ENDOSCOPE STRUCTURES AND TECHNIQUES FOR NAVIGATING TO A TARGET IN BRANCHED STRUCTURE, filed on April 16, 2003. Additional aspects of such a system relating to image registration and navigation are described in U.S. Patent No. 8,218,846, entitled AUTOMATIC PATHWAY AND WAYPOINT GENERATION AND NAVIGATION METHOD, filed on May 14, 2009; U.S. Patent Application Publication No. 2016/0000356, entitled REAL-TIME AUTOMATIC REGISTRATION FEEDBACK, filed on July 2, 2015; and U.S. Patent Application Publication No. 2016/0000302, entitled SYSTEM AND METHOD FOR NAVIGATING WITHIN THE LUNG, filed on June 29, 2015.

Document US 2017/319772 A1 discloses a system and method enabling the receipt of image data of a patient, identification of one or more locations within the image data depicting symptoms of lung disease, analyzing airways and vasculature proximate the identified locations, planning a pathway to the one or more locations, navigating an extended working channel to one of the locations, identifying the direction of blood flow within vasculature serving the location, positioning a catheter proximate the location, and temporarily isolating a portion of the identified vasculature serving the location depicting symptoms of lung disease.

In some cases, a bronchoscope may be too large to reach beyond the first few generations of airway branches or the CT images generated during the planning stages may not provide enough detail for the bronchoscope to reach a target tissue. Additionally, CT images may not represent a real-time depiction of the airways.

### SUMMARY

The invention is defined in the appended claims.

Existing challenges associated with the foregoing, as well as other challenges, are overcome by methods for facilitating bronchoscopy using ultrasound, and also by systems and computer-readable media that operate in accordance with the methods. In accordance with one aspect of the present disclosure, a method for facilitating electromagnetic navigation bronchoscopy using ultrasound is provided. The method includes receiving, from an electromagnetic sensor coupled to a distal portion of an extended working channel, an electromagnetic sensor signal value corresponding to a location, within a luminal network of a patient, of the distal portion of the extended working channel. Ultrasound image data is received from an ultrasound probe that protrudes from the distal portion of the extended working channel. Based on the ultrasound image data, an ultrasound image is displayed by way of a display device. An instruction to store location data corresponding to a location, within the luminal network of the patient, of target tissue is received by way of an input device. In response to the receiving of the instruction, the location data corresponding to the location of the target tissue is stored in a memory. The location data corresponding to the location of the target tissue is based on the received electromagnetic sensor signal value corresponding to the location of the distal portion of the extended working channel.

In another aspect of the present disclosure, the method further includes displaying, by way of the display device, a marker representing the location of the target tissue.

In a further aspect of the present disclosure, the method further includes receiving, from the electromagnetic sensor at a plurality of distinct times, a plurality of electromagnetic sensor signal values corresponding to a plurality of locations, within a luminal network of a patient, of the distal portion of the extended working channel at a respective one of the plurality of distinct times. A plurality of items of location data corresponding to the plurality of locations of the distal portion of the extended working channel at the plurality of distinct times, respectively, are stored in the memory. A plurality of markers representing the plurality of locations of the distal portion of the extended working channel, respectively, are displayed by way of the display device.

In still another aspect of the present disclosure, one of the plurality of electromagnetic sensor signal values corresponding to one of the plurality of locations of the distal portion of the extended working channel is stored when the one of the plurality of locations is a predetermined distance from a previously stored location of the distal portion of the extended working channel.

In yet another aspect of the present disclosure, the plurality of markers representing the locations of the extended working channel are displayed on the display device adjacent to the ultrasound image.

In another aspect of the present disclosure, the method further includes indicating, by way of the display device, that one of the plurality of markers corresponds to the location of the target tissue. In a further aspect of the present disclosure, the indicating includes changing an attribute of the one of the plurality of markers that corresponds to the location of the target tissue.

In still another aspect of the present disclosure, the attribute is a color, a size, or a pattern.

In yet another aspect of the present disclosure, the displaying of the ultrasound image includes displaying an ultrasound image that includes a representation of at least a portion of the target tissue, and the receiving of the instruction occurs concurrently with the displaying of the ultrasound image that includes the representation of the at least a portion of the target tissue.

In another aspect of the present disclosure, the method further includes determining a location of a distal portion of the ultrasound probe based on the electromagnetic sensor signal value corresponding to the location of the distal portion of the extended working channel.

In a further aspect of the present disclosure, the ultrasound probe protrudes a predetermined distance from the distal portion of the extended working channel, and the location of the ultrasound probe is determined based on the predetermined distance and the location of the distal portion of the extended working channel.

In still another aspect of the present disclosure, the method further includes receiving, from an additional electromagnetic sensor, coupled to the distal portion of the ultrasound probe, an additional electromagnetic sensor signal value corresponding to a location, within the luminal network of the patient, of the distal portion of the ultrasound probe. The determining of the location of the distal portion of the ultrasound probe is based on the additional electromagnetic sensor signal value.

In yet another aspect of the present disclosure, the method further includes determining the location of the target tissue relative to the location of the distal portion of the ultrasound probe, and generating the location data corresponding to the location of the target tissue based on the location of the target tissue relative to the location of the distal portion of the ultrasound probe.

In another aspect of the present disclosure, the method further includes processing the ultrasound image data, and determining, based on the processing of the ultrasound image data and the location of the distal portion of the ultrasound probe, the location of the target tissue within the luminal network of the patient.

In a further aspect of the present disclosure, the method further includes generating the location data corresponding to the location of the target tissue based on the electromagnetic sensor signal value corresponding to the location of the distal portion of the extended working channel at a time the instruction to store the electromagnetic sensor signal value corresponding to a location of target tissue is received.

In still another aspect of the present disclosure, the method further includes displaying, by way of the display device, a virtual target representing the target tissue.

In yet another aspect of the present disclosure, the method further includes generating an overlay representation of a location within the luminal network of the patient where a biopsy has been taken, and displaying the overlay upon a corresponding portion of the virtual target.

In another aspect of the present disclosure, the method further includes receiving, by way of the input device, an input indicating that a current location, within the luminal network of the patient, of the distal portion of the extended working channel corresponds to the location, within the luminal network of the patient, where the biopsy has been taken. In response to the receiving of the input, a location within the virtual target representing the location within the luminal network of the patient where the biopsy has been taken is identified.

In a further aspect of the present disclosure, the method further includes indicating, by way of the display device, a location within the virtual target where a biopsy needs to be taken.

In still another aspect of the present disclosure, an attribute of the virtual target displayed by way of the display device changes based on changes in the location of the distal portion of the extended working channel within the luminal network of the patient. In yet another aspect of the present disclosure, the attribute includes at least one of a size, a color, or a pattern of the virtual target.

In another aspect of the present disclosure, the method further includes receiving, by way of the input device, an input indicating the location of the target tissue on the ultrasound image displayed on the display device, and generating the location data corresponding to the location of the target tissue based on the received input indicating the location of the target tissue on the ultrasound image.

In a further aspect of the present disclosure, the display device includes a touch screen as the input device, and the input is a touch input received by way of a contact made between a user and the touch screen.

In accordance with another aspect of the present disclosure, a system for facilitating electromagnetic navigation bronchoscopy using ultrasound is provided. The system includes an ultrasound probe, an extended working channel configured to receive the ultrasound probe, a display device, an input device, and a computer. The extended working channel includes a distal portion on which an electromagnetic sensor is disposed. The computer includes a processor and a memory coupled to the processor. The memory has instructions stored thereon which, when executed by the processor, cause the computer to receive, from the electromagnetic sensor, an electromagnetic sensor signal value corresponding to a location, within a luminal network of a patient, of the distal portion of the extended working channel. Ultrasound image data is received from the ultrasound probe, which protrudes from the distal portion of the extended working channel. Based on the ultrasound image data, an ultrasound image is displayed by way of the display device. An instruction to store location data corresponding to a location, within the luminal network of the patient, of target tissue is received by way of the input device. In response to receipt of the instruction, the location data corresponding to the location of the target tissue is stored in the memory. The location data corresponding to the location of the target tissue is based on the received electromagnetic sensor signal value corresponding to the location of the distal portion of the extended working channel.

In accordance with another aspect of the present disclosure, a non-transitory computer-readable medium is described. The non-transitory computer-readable medium stores instructions that, when executed by a processor, cause the processor to perform a method for facilitating electromagnetic navigation bronchoscopy using ultrasound. The method includes receiving, from an electromagnetic sensor coupled to a distal portion of an extended working channel, an electromagnetic sensor signal value corresponding to a location, within a luminal network of a patient, of the distal portion of the extended working channel. Ultrasound image data is received from an ultrasound probe that protrudes from the distal portion of the extended working channel. Based on the ultrasound image data, an ultrasound image is displayed by way of a display device. An instruction to store location data corresponding to a location, within the luminal network of the patient, of target tissue is received by way of an input device. In response to the receiving of the instruction, the location data corresponding to the location of the target tissue is stored in a memory. The location data corresponding to the location of the target tissue is based on the received electromagnetic sensor signal value corresponding to the location of the distal portion of the extended working channel.

### BRIEF DESCRIPTION OF THE DRAWINGS

Various aspects and features of the present disclosure are described herein below with reference to the drawings, wherein:
FIG. 1 depicts a perspective view of an electromagnetic navigation system in accordance with the present disclosure;
FIG. 2 is a schematic diagram of an example computing device employed in the system of FIG. 1;
FIG. 3 is a flow diagram of an example method for identifying, navigating to, and performing a biopsy of a target tissue;
FIGS. 4A and 4B show a more detailed flow diagram of a portion of the example method of FIG. 3;
FIG. 5 illustrates an example user interface provided by way of the computing device of FIG. 2, presenting a view for navigation throughout a luminal network of a patient;
FIG. 6 is a more detailed flow diagram of a portion of the example method of FIG. 3; and
FIG. 7 illustrates an example user interface provided by way of the computing device of FIG. 2, presenting a view for performing a biopsy of target tissue.

### DETAILED DESCRIPTION

It would be beneficial to have improved EMN systems that are capable of assisting a clinician in identifying and navigating to target tissue, even in a case where the target tissue is located beyond the first few generations of the airway branches. For instance, it would be beneficial to employ ultrasound to assist in identifying and confirming the location of the bronchoscope and navigate to target tissue. One technical challenge in doing so, however, is that, because an ultrasound probe is capable of imaging tissue only within a finite distance from the probe itself (for example, air in the lungs may prevent the ultrasound probe from detecting a lesion or may otherwise limit the distance at which the ultrasound probe can detect a lesion) and the direction the ultrasound probe is imaging may be unknown, searching airways for target tissue can become laborious and, in some cases, may involve unintentionally searching particular airways multiple times, thus reducing the speed and efficiency with which the target tissue can be located.

This disclosure is related to systems, methods, and computer-readable media for facilitating electromagnetic navigation bronchoscopy using ultrasound. As will be appreciated in view of this disclosure, the systems, methods, and computer-readable media described herein facilitate location of and/or navigation to target tissue and the performing of a biopsy of the target tissue with improved efficiency and effectiveness, even in cases where target tissue is located beyond the first few generations of the airway branches. In general, the various embodiments described herein employ an ultrasound probe to identify and navigate to a target tissue. Despite ultrasound probes generally being capable of imaging tissue only within a finite distance from the probes themselves, the embodiments described herein avoid the need to conduct laborious and repetitive searching of airways for target tissue, and thereby improve the speed and efficiency with which the target tissue can be located. Additionally, the various embodiments described herein facilitate improved accuracy of biopsy procedures by supplementing electromagnetic navigation bronchoscopy with an ultrasound. In particular, ultrasound is used in cooperation with electromagnetic navigation bronchoscopy to confirm the location of an extended working channel of the bronchoscope. Particular embodiments of this disclosure are described below with reference to the accompanying drawings.

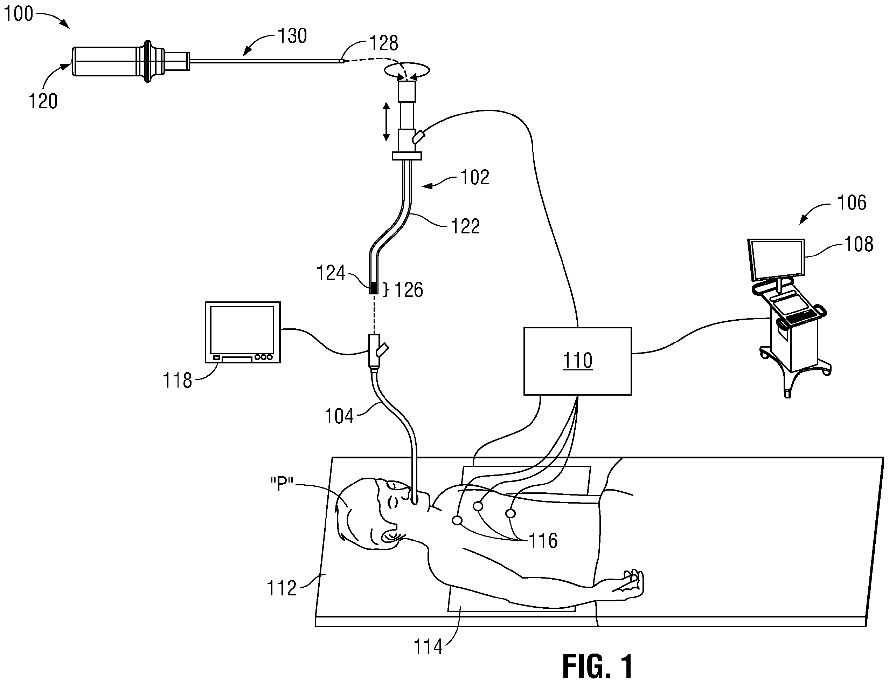

FIG. 1 illustrates an example electromagnetic navigation (EMN) system 100 provided in accordance with this disclosure. In general, the EMN system 100 is configured to identify a location and/or an orientation of a medical device being navigated toward a target location within a patient's body. In some cases, the EMN system 100 is further configured to augment computed tomography (CT) images, magnetic resonance imaging (MRI) images, fluoroscopic images, and/or ultrasonic images employed during navigation of the medical device through the patient's body toward a target of interest, such as a deceased portion of tissue in a luminal network of the patient's lung.

The EMN system 100 includes a catheter guide assembly 102, a bronchoscope 104, a computing device 106, a display device 108, a tracking device 110, a patient platform 112, antenna assembly 114, reference sensors 116, a monitoring device 118, and an ultrasound probe 120. The bronchoscope 104 is operatively coupled to the computing device 106 (by way of the tracking device 110) and the monitoring device 118 via respective wired connections (as shown in FIG. 1) or wireless connections (not shown in FIG. 1). During a navigation phase of an EMN bronchoscopy procedure, the bronchoscope 104 is inserted into the oral cavity of a patient "P" and captures images of the luminal network of the patient "P's" lung. The catheter guide assembly 102 is inserted into the bronchoscope 104 to access the periphery of the luminal network of the lung of the patient "P." The catheter guide assembly 102 includes a catheter or extended working channel (EWC) 122 with an EM sensor 124 affixed to a portion, for example, a distal portion 126, of the EWC 122. The EM sensor 124 is communicatively coupled to the tracking device 110 by way of one or more wired or wireless communication paths. For instance, in some embodiments, the EM sensor 124 is communicatively coupled to the tracking device 110 by way of one or more wires 132 that protrude from a port of the catheter guide assembly 102. In some examples, at least a portion (which is not explicitly shown in FIG. 1) of the one or more wires 132 (including a portion where the one or more wires 132 are coupled to the EM sensor 124) is internal to, included as a part of, and/or otherwise affixed to, the catheter guide assembly 102. The EM sensor 124 is configured to receive a signal based on an electromagnetic field radiated by the antenna assembly 114, provide the received signal to the tracking device 110, which uses the received signal to determine a location and/or an orientation of the EM sensor 124 and, thus, the location of the distal portion 126 of EWC 122 during navigation through the luminal network of the lung. Although the context of the present embodiment is one in which EM sensor 124 is affixed to a portion of the EWC 122, other embodiments without the EWC 122 are also envisioned, such as where the EM sensor 124 is affixed to a distal portion of the bronchoscope 104 itself.

Due to its size, the bronchoscope 104 is limited in how far it can travel through the periphery of the luminal network of the lung of the patient "P." Thus, the EWC 122 of the catheter guide assembly 102 is inserted into the bronchoscope 104 to access the periphery of the lungs. To assist in visualizing and navigating the periphery of the lungs, an ultrasound probe 120 is inserted into the catheter guide assembly 102 and EWC 122. Ultrasound probe 120 may be any number of types of endobronchial ultrasound probes suitable for use in a bronchoscope 104 and/or a catheter guide assembly 102. For example, in embodiments, ultrasound probe 120 may be a radial ultrasound, a linear ultrasound, or a convex ultrasound. The ultrasound probe 120 includes a proximal portion 130 and a distal portion 128. The distal portion 128 of the ultrasound probe 120 protrudes past the distal portion 126 of the EWC 122 to aid in visualizing the surrounding area of the distal portion 126 of the EWC 122.

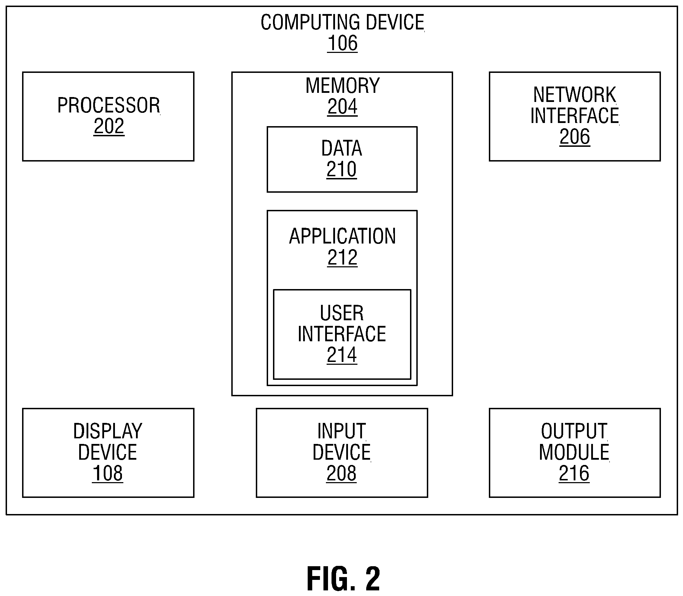

Before continuing to describe the EMN system 100 illustrated in FIG. 1, reference will be made to FIG. 2, which shows example aspects of the computing device 106 of the system 100. The computing device 106 is generally configured to execute the various functions of the procedures described herein. Additionally, in some embodiments, instead of including a tracking device 110 that is separate from, and communicatively coupled to, the computing device 106, the functions and/or procedures of the tracking device 110 are implemented by the computing device 106. The computing device 106, which, in various embodiments, may be a laptop, desktop, tablet, or any other suitable computing device, includes a display device 108, one or more processors 202, one or more memories 204, a network interface 206, one or more input devices 208 and one or more output modules 216. Memory 204 includes any non-transitory computer-readable storage media for storing data, instructions, and/or other types of software that is executable by processor 202 and which controls the operation of computing device 106. In an embodiment, memory 204 may include one or more solid-state storage devices such as flash memory chips. Alternatively, or in addition to the one or more solid-state storage devices, memory 204 may include one or more mass storage devices connected to the processor 202 through a mass storage controller (not shown in FIG. 2) and a communications bus (not shown in FIG. 2).

Although the description of computer-readable media contained herein refers to a solid-state storage, it should be appreciated by those skilled in the art that computer-readable storage media can be any available media that can be accessed by the processor 202. That is, computer readable storage media includes non-transitory, volatile and non-volatile, removable and non-removable media implemented in any method or technology for storage of information such as computer-readable instructions, data structures, program modules or other data. For example, computer-readable storage media includes RAM, ROM, EPROM, EEPROM, flash memory or other solid-state memory technology, CD-ROM, DVD, Blu-Ray or other optical storage, magnetic cassettes, magnetic tape, magnetic disk storage or other magnetic storage devices, or any other medium which can be used to store the desired information and which can be accessed by computing device 106.

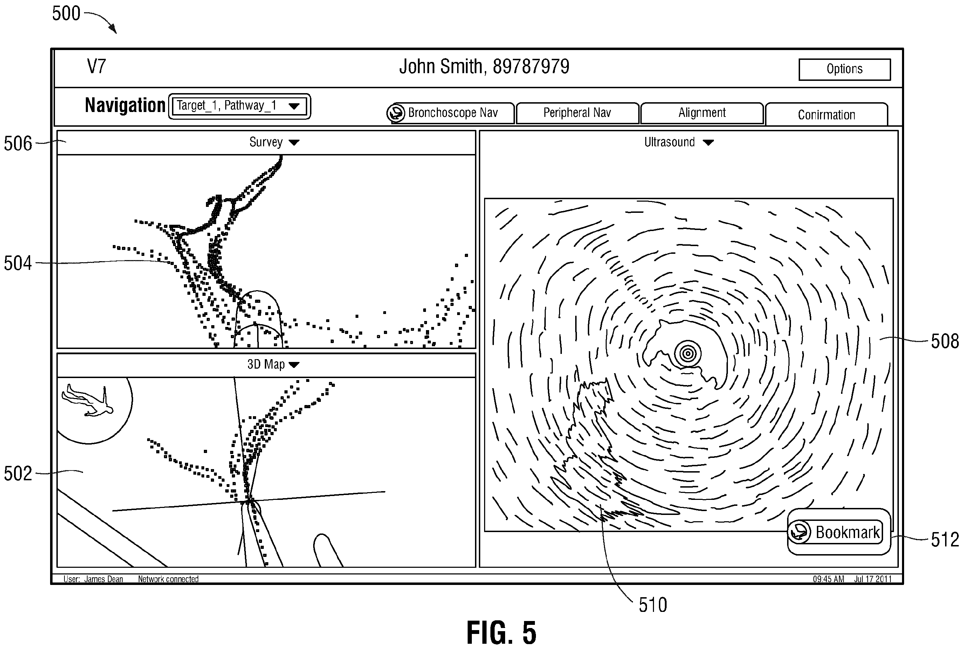

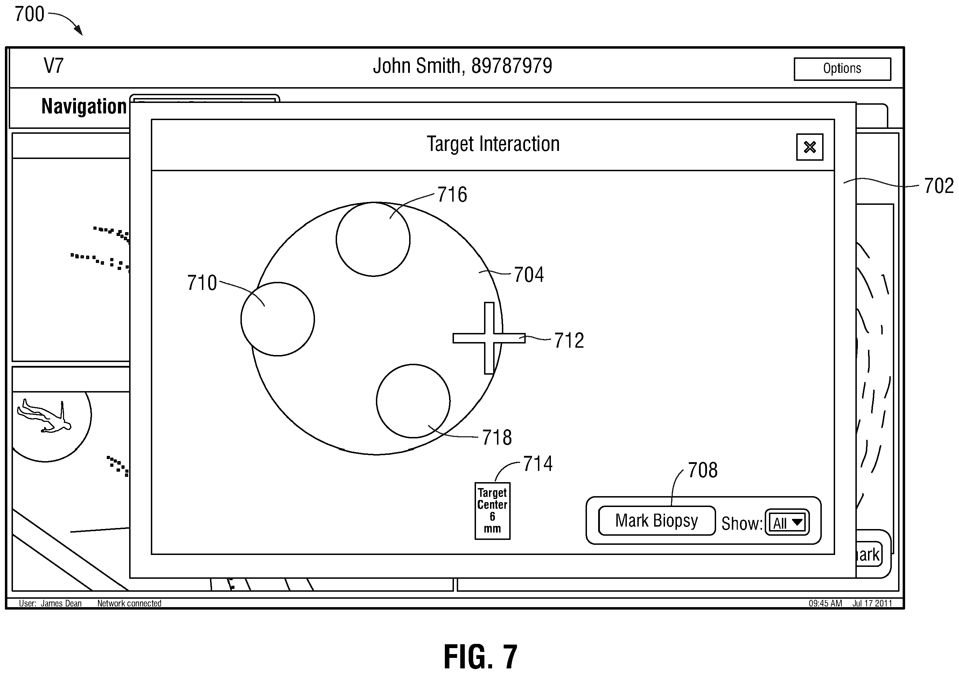

Memory 204 may store application 212 and/or data 210, for example, image data, location data, and/or other types of data. Application 212 may include user interface instructions 214 that, when executed by the processor 202, cause the display device 108 to present one or more user interfaces, such as, for example, the example user interface 500 illustrated in FIG. 5 and/or the example user interface 700 illustrated in FIG. 7. Network interface 206 may be configured to connect to a network such as a local area network (LAN) consisting of a wired network and/or a wireless network, a wide area network (WAN), a wireless mobile network, a Bluetooth network, and/or the internet. Input device 208 may be any device by means of which a clinician may interact with computing device 106, such as, for example, a mouse, a keyboard, a foot pedal, a touch screen, and/or a voice interface. Output module 216 may include any connectivity port or bus, such as, for example, parallel ports, serial ports, universal serial busses (USB), or any other similar connectivity port known to those skilled in the art.

The particular configuration of the computing device 106 illustrated in FIG. 2 is provided as an example, but other configurations of the components shown in FIG. 2 as being included in the computing device 106 are also contemplated. In particular, in some embodiments, one or more of the components shown in FIG. 2 as being included in the computing device 106 may instead be separate from the computing device 106 and may be coupled to the computing device 106 and/or to any other component(s) of the EMN system 100 by way of one or more respective wired or wireless path(s) to facilitate the transmission of power and/or data signals throughout the EMN system 100.

In some aspects, the EMN system 100 may also include multiple computing devices 106, wherein the multiple computing devices 106 are employed for planning, treatment, visualization, or helping clinicians in a manner suitable for medical operations. The display device 108 may be touch-sensitive and/or voice-activated, enabling the display device 108 to serve as both an input device and an output device. The display device 108 may display two-dimensional (2D) images or three-dimensional (3D) images, such as a 3D model of a lung, to enable a practitioner to locate and identify a portion of the lung that displays symptoms of lung diseases. The display device 108 may also display ultrasound images received from the ultrasound probe 120.

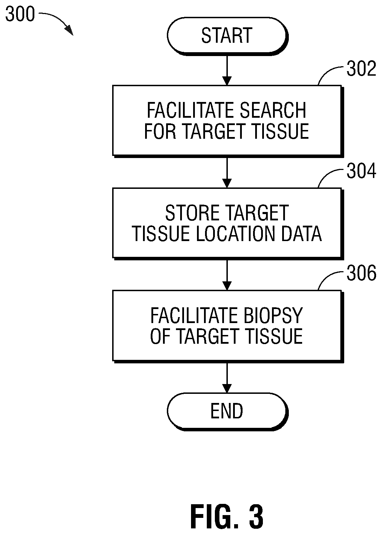

The one or more memories 204 store one or more programs and/or computer-executable instructions that, when executed by the one or more processors 202, cause the one or more processors 202 to perform various functions and/or procedures, such as, for instance, the procedures described herein in connection with FIG. 3, FIG. 4A, FIG. 4B, and/or FIG. 6. For example, the processors 202 may calculate a location and/or an orientation of the EM sensor 124 based on the electromagnetic signal that is radiated by the antenna assembly 114 and received by the EM sensor 124. The processors 202 may also perform image-processing functions to cause the 3D model of the lung to be displayed on the display device 108 or cause the ultrasound image received from the ultrasound probe 120 to be displayed on the display device 108. The processors 202 may also generate one or more electromagnetic signals to be radiated by way of the antenna assembly 114. In some embodiments, the computing device 106 may further include a separate graphic accelerator (not shown) that performs only the image-processing functions so that the one or more processors 202 may be available for other programs. The one or more memories 204 also store data 210, such as mapping data for EMN, image data, patients' medical record data, prescription data, and/or data regarding a history of the patient's diseases, and/or other types of data.

Referring now back to FIG. 1, the patient platform 112 is configured to provide a flat surface upon which the patient "P" lies during the EMN navigation procedure. The antenna assembly 114, which may also be referred to as an EM field-generating device, is arranged upon the platform 112 or is included as a component of the platform 112. The antenna assembly 114 includes one or more antennas (not shown in FIG. 1). With the patient "P" lying upon the platform 112, the one or more processors 202 (or another signal generator not shown in FIG. 1) generate and provide to the antenna(s) of the antenna assembly 114 one or more AC current signals that the antenna(s) convert into one or more respective EM signal(s) and radiate in a manner sufficient to overlap with a volume occupied by the patient "P." In this manner, EMN system 100 generates an electromagnetic field that allows for the tracking of the position of the EM sensor 124 within the generated electromagnetic field.

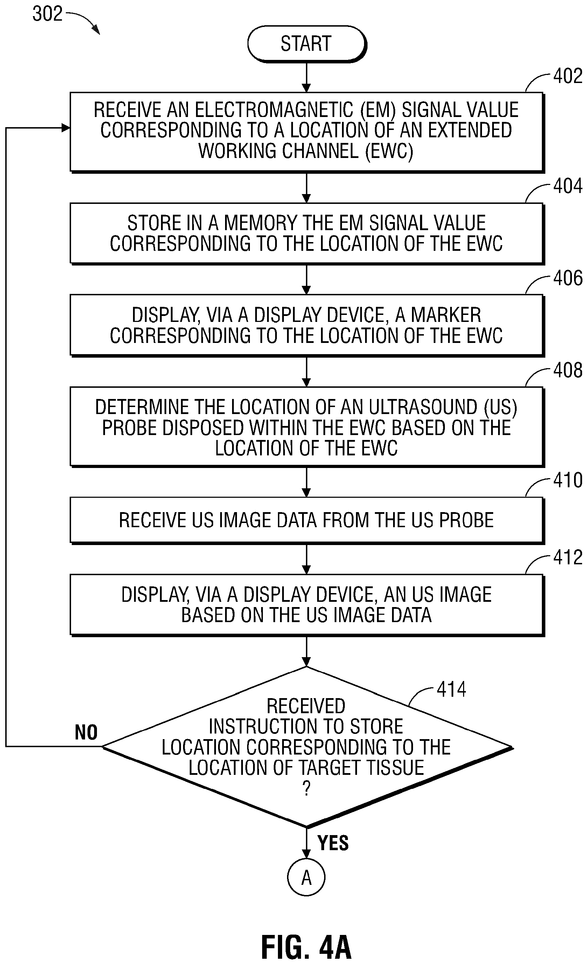

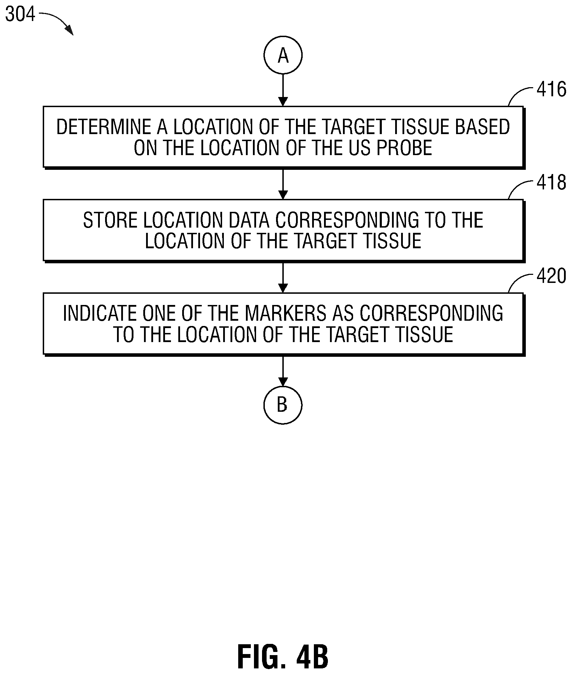

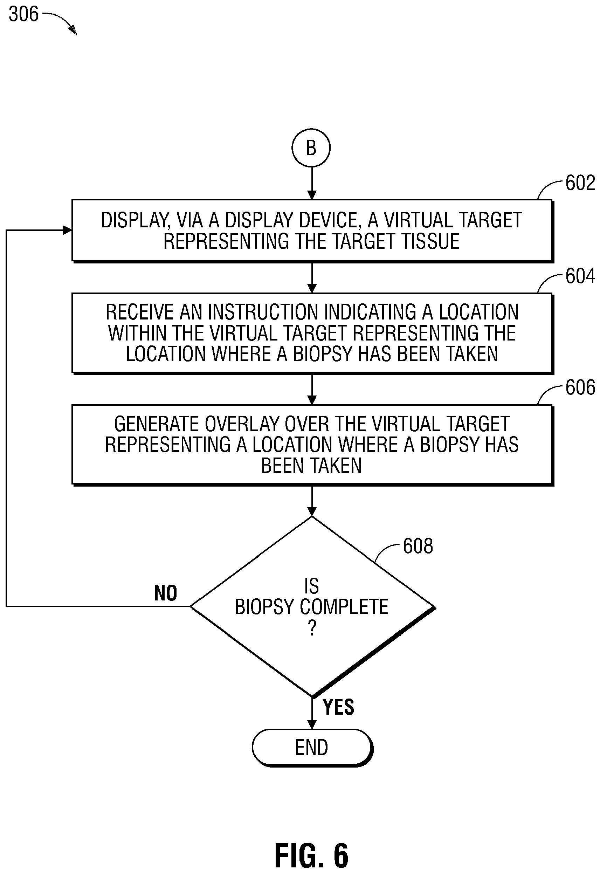

Having described aspects of the EMN system 100 with reference to FIG. 1 and FIG. 2, reference will now be made to FIG. 3, FIG. 4A, FIG. 4B, and FIG. 6 to describe aspects of an example procedure 300 for utilizing the system 100 to identify, navigate to, and/or perform a biopsy of, a target tissue. Before describing particular details of the procedure 300 in the context of FIG. 4A, FIG. 4B, and FIG. 6, a general overview of the procedure 300 will be provided in the context of FIG. 3. In general, the procedure 300 includes three phases-a target tissue search phase (block 302), a target tissue location data storage phase (block 304), and a biopsy phase (block 306). The procedure 300 begins at block 302 with a search for a target tissue. Once the bronchoscope 104 and the catheter guide assembly 102 are inserted into the oral cavity of the patient "P," the ultrasound probe 120 is inserted into the EWC 122 of the catheter guide assembly 102. Using the EM sensor 124 disposed on the distal portion 126 of EWC 122, the location of the distal portion 126 of the EWC 122, and, by extension, the locations of the EWC 122 and the ultrasound probe 120 can be tracked. Ultrasound probe 120 is used to perform a more exact search in the periphery of the bronchial tree. Specifically, ultrasound probe 120 can be used to generate an ultrasound image (for example, the ultrasound image 508 described below) of the periphery of the bronchial tree that is presented to the clinician by way of the display device 108. The clinician observes the generated ultrasound images while repositioning the ultrasound probe 120 within the luminal network of the patient "P" to locate the target tissue within the luminal network. By utilizing the tracked location of the EM sensor 124, the clinician can perform a more efficient search of the periphery of the bronchial tree. In particular, the tracking of the EM sensor 124 informs the clinician of what areas of the periphery of the bronchial tree is currently being searched and what areas have already been searched. This ensures that the clinician does not search the same area multiple times and ensures that the search is done in the vicinity of the target tissue.

At block 304, once the target tissue has been reached and identified by using the ultrasound probe 120, location data corresponding to the location of the target tissue is stored in the memory 204 of the computing device 106 for subsequent use by the clinician. As will be described in further detail below, the location data stored at block 304 is based on a received electromagnetic sensor signal value corresponding to a location of the distal portion 126 of the EWC 122. In particular, at block 304, using the location of the EM sensor 124 disposed on the distal portion 126 of the EWC 122, as determined by the tracking device 110 and/or the computing device 106, the location of the distal portion 128 of the ultrasound probe 120, and thus the location of the target tissue, is determined by the tracking device 110 and/or the computing device 106. Once the location data has been stored at block 304, the stored location data is used during a subsequent phase of the procedure, for instance, to facilitate accurate navigation to the target tissue during a tool exchange, whereby the ultrasound probe 120 is removed from the patient "P" and is replaced with a biopsy tool (not shown in FIG. 1), and/or during a biopsy phase of the procedure conducted at block 306.

The final stage of the procedure 300, depicted in block 306, is the management of the biopsy procedure of the target tissue. Once the location of the target tissue has been determined and/or the location data has been stored at block 304, a biopsy tool (not shown in FIG. 1) is inserted into the luminal network of the patient "P" by way of the EWC 122 to extract a portion of the target tissue, which is to be subsequently tested for various characteristics. As described in further detail below, the computing device 106 utilizes the previously acquired ultrasound image of the target tissue to render a virtual target to assist in the biopsy procedure of the target tissue.

Having provided a general overview of the procedure 300 in the context of FIG. 3, more detailed aspects of the procedure 300 will now be described with reference to FIGS. 4A-7. In particular, flow diagrams of FIG. 4A, FIG. 4B, and FIG. 6 illustrates more detailed aspects of the target tissue search phase (block 302 of FIG. 3), the target tissue location data storage phase (block 304 of FIG. 3), and the biopsy phase (block 306 of FIG. 3), respectively, of the procedure 300. FIG. 5 illustrates an example user interface 500 provided by way of the computing device 106 during the target tissue search phase (block 302 of FIG. 3) and target tissue location data storage phase (block 304 of FIG. 3) of the procedure 300, and FIG. 7 illustrates an example user interface 700 provided by way of the computing device 106 during the biopsy phase (block 306 of FIG. 3) of the procedure 300.

Although not shown in FIG. 4A, prior to block 402, computing device 106 receives CT scan data of a luminal network of the patient "P." Utilizing the CT scan data, computing device 106 generates a 3D model 502 (depicted in the user interface 500 of FIG. 5) corresponding to the luminal network of the patient "P". The 3D model 502 is displayed to the clinician via the user interface 500 of display device 108. Computing device 106 also utilizes the 3D model 502 to create a planned pathway through the luminal network of the patient "P" to the target tissue that, together with the generated 3D model, is employed by the clinician to assist in navigation throughout the luminal network of the patient "P."

Patient "P" is then placed on antenna assembly 114, which generates one or more electromagnetic fields that are sensed by reference sensors 116 and the EM sensor 124 affixed to the EWC 122. The computing device 106 then indicates to the clinician, by way of the 3D model 502, a suggested path within the luminal network of the patient "P" along which to navigate the EWC 122 to arrive at the target tissue. To begin, a bronchoscope 104 is inserted into the oral cavity of the patient "P." The EWC 122 is then inserted into the bronchoscope 104. At block 402, computing device 106 receives, from EM sensor 124 coupled to the distal portion 126 of the EWC 122, an EM sensor 124 signal value corresponding to a location, within the luminal network of the patient "P," of the distal portion 126 of the EWC 122.

At block 404, computing device 106 stores, in memory 204, the EM sensor 124 signal value, which was received at block 402, and which corresponds to the location of the distal portion 126 of the EWC 122. Thus, as the EWC 122 is navigated through the luminal network of the patient "P", computing device 106 continually or periodically tracks and stores data indicating the historical locations of the distal portion 126 of the EWC 122 at various times during navigation, and thus indicating the pathway that the EWC 122 has traveled within the luminal network.

At block 406, the display device 108 displays, by way of the user interface 500, one or more markers 504, each of the markers 504 corresponding to one of the locations of the distal portion 126 of the EWC 122 for which corresponding data was stored at block 404. In particular, as depicted in FIG. 5, markers 504 are displayed on user interface 500 in a survey window 506 to depict the pathways in the luminal network of the patient "P" that the EWC 122 has already traveled during the target tissue search phase (block 302 of FIG. 3). In some embodiments, the markers 504 are superimposed over the 3D model 502 corresponding to the luminal network of the patient "P." In this manner, the clinician is provided, by way of the user interface 500, with a continually updated indication of the particular paths within the luminal network of the patient "P" that the EWC 122 has already traversed, thereby enabling the clinician to avoid searching particular paths multiple times, thus improving the speed and efficiency with which the target tissue can be located. In some examples, the computing device 106 stores in the memory 204 a timestamp associated with each of the EM sensor 124 signal values corresponding to a time the EM sensor 124 signal values were received.

At block 408, with the ultrasound probe 120 inserted into the EWC 122 such that the distal portion 128 of the ultrasound probe 120 protrudes from the distal portion 126 of the EWC 122, the location of the distal portion 128 of the ultrasound probe 120 is determined based on the location of the distal portion 126 of the EWC 122. The location of the distal portion 128 of the ultrasound probe 120 is determined in a number of different ways, in accordance with various embodiments. In one embodiment, the distal portion 128 of the ultrasound probe 120 protrudes from the distal portion 126 of the EWC 122 by a known distance. In this embodiment, for example, the ultrasound probe 120 locks to the EWC 122 at a distal portion and/or a proximal portion of the ultrasound probe 120 and the EWC 122 (not shown in FIG. 1). Thus, once the location of the distal portion 126 of the EWC 122 is determined in the manner described above, the location of the distal portion 128 of the ultrasound probe 120 is determined based on the known distance by which the distal portion 128 of the ultrasound probe 120 protrudes from the distal portion 126 of the EWC 122. In another embodiment, the EWC 122 has a known length and the ultrasound probe 120 has hash marks (not shown in FIG. 1) disposed, along the proximal portion 130 of the ultrasound probe 120, at known and/or marked distances from the distal end of the ultrasound probe 120. Thus, as the ultrasound probe 120 is pushed past a distal portion 126 of the EWC 122, the location of the distal portion 128 of the ultrasound probe 120 relative to the EM sensor 124 disposed at a distal portion 126 of the EWC 122 can be determined by measuring the visible hash marks. In yet another embodiment, an additional EM sensor (not shown in FIG. 1) is coupled to the distal portion 128 of the ultrasound probe 120, and the computing device 106 receives from the additional EM sensor an additional EM sensor signal value corresponding to a location, within the luminal network of a patient "P," of the distal portion 128 of the ultrasound probe 120.

At block 410, computing device 106 receives ultrasound image data from the ultrasound probe 120. The computing device 106 processes the ultrasound image data and, based on the received ultrasound image data, displays at block 412, via display device 108, an ultrasound image 508 (FIG. 5) of the region of the luminal network of the patient "P" where the distal portion 128 of the ultrasound probe 120 is located. Display of the ultrasound image 508 allows the clinician to inspect various portions of tissue of the luminal network, including, for example, tissue located at a target site and/or tissue located elsewhere in the luminal network.

When the ultrasound probe 120 reaches the target tissue, an ultrasound image including an ultrasound image of a portion of the target tissue 510 is displayed in the ultrasound image 508. When the clinician observes the ultrasound image including the portion of the target tissue 510 in the ultrasound image 508, the clinician provides to the computing device 106, by way of the input device 208, an instruction (also referred to herein as a storage instruction) to store location data corresponding to the location, within the luminal network of the patient "P", of the EM sensor 124, while the target tissue 510 remains displayed in the ultrasound image 508. The location data that corresponds to the location, within the luminal network of the patient "P", of the EM sensor 124 while the target tissue 510 remains displayed in the ultrasound image 508 corresponds to the location of the target tissue 501. In various embodiments, the clinician may instruct the computing device 106 to store the location data in a number of different ways. For example, in a case where the display device 108 includes a touch screen that functions as the input device 208, the clinician can instruct the computing device 106 to store the location data by selecting a bookmark button 512 (FIG. 5) displayed on the user interface 500. Alternatively, in a case where the input device 208 is a foot pedal or a computer mouse, the clinician may instruct the computing device 106 to store the location data by actuating the foot pedal and/or the computer mouse. These examples of types of input devices 208 by which the clinician may provide instructions to the computing device 106 are merely provided by way of illustration, not limitation. In other embodiments, any suitable type of input device 208 may be used by the clinician to provide instructions to the computing device 106.

At block 414, a determination is made as to whether the storage instruction is received by way of the input device 208. If it is determined at block 414 that the storage instruction has not been received, for instance, indicating that the target tissue has not yet been reached, then the procedures of blocks 402-412 are repeated. In this manner, blocks 402-412 are continually repeated as the EWC 122 and the ultrasound probe 120 are navigated throughout the luminal network of the patient "P." As the EWC 122 is moved a predetermined distance from a previously stored location, a new EM sensor 124 signal value corresponding to a new location of the EWC 122 is stored in the memory 204 by the computing device 106. If, on the other hand, it is determined at block 414 that the storage instruction has been received by way of the input device 208, the procedure progresses to block 416.

In various embodiments, the location data for which the storage instruction was received at block 414, can be any type of location data that enables the clinician to navigate a tool back to the target tissue 510, for example, after a tool exchange. In each embodiment, the location data generally corresponds to the location of the target tissue within the luminal network of the patient "P". In some embodiments, the location data indicates a location, within the luminal network of the patient "P", of the EM sensor 124, the distal portion 126 of the EWC 122, and/or the distal portion 128 of the ultrasound probe 120, as determined at a time when the EM sensor 124, the distal portion 126 of the EWC 122, and/or the distal portion 128 of the ultrasound probe 120 are positioned, within the luminal network of the patient "P", proximal to the target tissue.

In another embodiment, the location data indicates a location, within the luminal network of the patient "P", of the target tissue itself. In this embodiment, at block 416, the location, within the luminal network of the patient "P", of the target tissue 510 is determined. In particular, the location of the target tissue 510 relative to the EM sensor 124 and the distal portion 128 of the ultrasound probe 120 is determined. In embodiments, the location data indicating the location of the target tissue 510 is utilized by the computing device 106 to update the registration and location of the target tissue obtained in a planning stage. In embodiments, the location data corresponding to the location of the target tissue 510 relative to the ultrasound probe 120 is compared with the EM sensor 124 signal value corresponding to the location of the distal portion 126 of the EWC 122. The distance between the location of the distal portion 126 of the EWC 122 and the location of the target tissue 510 is determined based on a result of this comparison. The computing device 106 generates location data corresponding to the location of the target tissue 510 to be stored in the memory 204. At block 418, the location data for which the storage instruction was received at block 414 is stored in the memory 204.

At block 420, the location data that was stored at block 418 and that corresponds to the location of the target tissue 510 is associated with a corresponding marker, such as, for example marker 504 (FIG. 5). In some embodiments, the location of the target tissue 510 is digitally stored and in other embodiments, the location of the target tissue 510 is digitally stored and also displayed by way of the display device 108 via a corresponding marker. Although not shown in FIG. 5, in some embodiments, the marker 504 associated with the location of the target tissue is a different shape, color, or pattern than other ones of the markers 504 to allow a clinician to easily identify which marker 504 corresponds to the location of the target tissue. As depicted in FIG. 5, the ultrasound image 508 is displayed adjacent the survey window 506. However, other configurations are also envisioned. For example, the markers 504 may be superimposed over the ultrasound image 508 and/or the 3D model 502.

Once the target tissue search phase (block 302 of FIG. 3) and target tissue location data storage phase (block 304 of FIG. 3) have been completed, the procedure 300 proceeds to the biopsy phase (block 306 of FIG. 3), which is described in further detail in connection with FIG. 6 and FIG. 7, and in which the clinician performs a biopsy of the target tissue 510 with the assistance of the system 100. In particular, to facilitate the biopsy, a tool exchange is performed in which the clinician removes the ultrasound probe 120 from the EWC 122 in order to navigate the biopsy tool (not shown in FIG. 1) to the target tissue. Since the biopsy is performed without the aid of a live ultrasound image from the ultrasound probe 120, when a clinician begins the biopsy of the target tissue, the computing device 106 displays a user interface 700 including a biopsy screen 702 via the display device 108 at block 602 to assist the clinician in performing the biopsy. In particular, the computing device 106 generates a virtual target 704, which is displayed via the biopsy screen 702, and which represents the target tissue. In some embodiments, the virtual target 704 is generated by mapping a predetermined set of locations within the luminal network of the patient "P" to a geometrical shape. In other embodiments, the computing device 106 performs image processing of the ultrasound image 508 including the ultrasound image of the portion of the target tissue 510 to generate a virtual target 704. In some examples, the computing device 106 automatically displays biopsy screen 702 and virtual target 704 on the display device 108 when the distal portion 136 of the EWC 122 is navigated to within a predetermined distance, for example, from about 1 cm to about 5 cm, from the location indicated by the location data that was stored at block 418 and that corresponds to the location of the target tissue.

At block 604, once a biopsy has been taken by the clinician, by extracting a portion of the target tissue, the clinician provides to the computing device 106, by way of the input device 208, an input indicating a portion of the virtual target 704 that corresponds to the portion of the target tissue where the biopsy was taken. In some embodiments, the exact direction of the target tissue cannot be determined, therefore, the virtual target 704 may correspond to portions of the pathway that are targets for biopsy locations. Therefore, a user is aided to ensure that a biopsy has been taken in all directions, thereby increasing the likelihood that a biopsy of the target tissue is acquired. In various embodiments, the input provided by the clinician can be provided by any type of the input device 208, such as a computer mouse, a touch screen device, and/or the like, together with a selection of the "Mark Biopsy" button 708, for instance.

The biopsy screen 702 allows the clinician to indicate on the biopsy screen 702 the portion of the virtual target 704 that corresponds to the portions of the target tissue at which the clinician has taken the biopsy. At block 606, the computing device 106 generates an overlay 710 indicating the portion of the virtual target 704 that corresponds to the portion of the target tissue at which the biopsy has been taken by the clinician. As the clinician extracts subsequent biopsy samples at other portions of the target tissue, the clinician provides additional inputs to the computing device 106 indicating the portions of the virtual target 704 that correspond to the portions of the target tissue where the biopsy portions have been extracted. The computing device generates additional overlays, such as overlays 716 and 718, indicating the additional portions of the virtual target 704 that correspond to the portions of the target tissue at which the biopsy samples have been extracted by the clinician. In this manner, the clinician may keep track of which portions of the target tissue have been biopsied, to ensure a thorough and accurate biopsy yield is obtained. Thus, the accuracy of the biopsy procedure may be improved, despite the orientation of the target tissue with respect to the ultrasound probe 120 possibly remaining unknown.

In some embodiments, an attribute of the virtual target 704 and/or the overlays 710, 716, 718 changes based on the location of the distal portion 126 of the EWC 122 within the luminal network of the patient "P." In other embodiments, the size of the displayed virtual target 704 and/or the overlays 710, 716, 718 changes when the EWC 122 is closer to the target tissue 510. For example, the virtual target 704 and/or the overlays 710, 716, 718 is smaller when the location of the distal portion 126 of the EWC 122 is farther from the target tissue 510, and vice versa, is larger when the location of the distal portion 126 of the EWC 122 is closer to the target tissue 510.

At block 608, if the biopsy is incomplete (for instance, if any locations within the virtual target 704 remain where a biopsy still needs to be taken), the functions of blocks 602-606 are repeated. If, on the other hand, no locations within the virtual target 704 where a biopsy needs to be taken remain, the biopsy is marked complete. In some embodiments, when no locations where a biopsy needs to be taken remain, an indicator (not shown in FIG. 7) is displayed, by way of the display device 108. The indicator, in various embodiments, can be a textual message, an audible sound, and/or any other suitable indicator. Biopsy screen 702, in some embodiments, further includes an indicator 712 that represents the location of the distal portion 126 of the EWC 122 relative to the target tissue 510. In embodiments, the indicator is a crosshair and moves relative to the location of the distal portion 126 of the EWC 122. Biopsy screen 702 also includes, in some examples, a distance indicator 714 which displays the distance between the distal portion 126 of the EWC 122 and the approximate center of the target tissue 510.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting but merely as exemplifications of particular embodiments. Those skilled in the art will envision other modifications within the scope of the claims appended hereto.

## Claims

1. A system for facilitating electromagnetic navigation bronchoscopy using ultrasound, comprising:
an ultrasound probe;
a catheter configured to receive the ultrasound probe, the catheter including a distal portion on which an electromagnetic sensor is disposed;
a display device;
an input device; and
a computer including:
a processor; and
a memory coupled to the processor, the memory having instructions stored thereon which, when executed by the processor, cause the computer to:
(i) repeatedly, while the catheter and the ultrasound probe inserted therein are navigated throughout a luminal network of a patient, and until an instruction to store location data corresponding to a location of target tissue is received:
receive, from the electromagnetic sensor, an electromagnetic sensor signal value corresponding to a location of the distal portion of the catheter within the luminal network of a patient;
store in the memory the plurality of received electromagnetic sensor signal values;
display, on the display device in a survey window of a user interface, a marker indicating the location of the distal portion of the catheter;
determine a location of a distal portion of the ultrasound probe based on the electromagnetic sensor signal value corresponding to the location of the distal portion of the catheter and a known distance by which the distal portion of the ultrasound probe protrudes from the distal portion of the catheter;
receive ultrasound image data from the ultrasound probe, wherein the ultrasound probe protrudes from the distal portion of the catheter; and
display, by way of the display device, an ultrasound image based on the received ultrasound image data;
(ii) once an instruction to store location data corresponding to a location of target tissue, while at least a portion of the target tissue is shown in the displayed ultrasound image, is received by way of the input device, store the location data corresponding to the location of the target tissue in the memory, wherein the location data corresponding to the location of the target tissue is based on the received electromagnetic sensor signal value corresponding to the location of the distal portion of the catheter, while the at least a portion of the target tissue is shown in the displayed ultrasound image.

2. The system according to claim 1, wherein the memory has further instructions stored thereon which, when executed by the processor, cause the computer to display, by way of the display device, the survey window adjacent to the ultrasound image.

3. The system according to claim 1, wherein the plurality of electromagnetic sensor signals values are received at a plurality of distinct times.

4. The system according to claim 1, wherein one of the plurality of electromagnetic sensor signal values corresponding to one of the plurality of locations of the distal portion of the catheter is stored when the one of the plurality of locations is a predetermined distance from a previously stored location of the distal portion of the catheter.

5. The system according to any preceding claim, wherein the memory has further instructions stored thereon which, when executed by the processor, cause the computer to receive, from an additional electromagnetic sensor, coupled to the distal portion of the ultrasound probe, an additional electromagnetic sensor signal value corresponding to a location, within the luminal network of the patient, of the distal portion of the ultrasound probe, wherein the determining of the location of the distal portion of the ultrasound probe is based on the additional electromagnetic sensor signal value.

6. The system according to any preceding claim, wherein the memory has further instructions stored thereon which, when executed by the processor, cause the computer to determine the location of the target tissue relative to the location of the distal portion of the ultrasound probe; and
to generate the location data corresponding to the location of the target tissue based on the location of the target tissue relative to the location of the distal portion of the ultrasound probe; and/or cause the computer further to process the ultrasound image data; and
determine, based on the processing of the ultrasound image data and the location of the distal portion of the ultrasound probe, the location of the target tissue within the luminal network of the patient.

7. The system according to any preceding claim, wherein the memory has further instructions stored thereon which, when executed by the processor, cause the computer to generate the location data corresponding to the location of the target tissue based on the electromagnetic sensor signal value corresponding to the location of the distal portion of the catheter at a time the instruction to store the electromagnetic sensor signal value corresponding to a location of target tissue is received.

8. The system according to any preceding claim, wherein the memory has further instructions stored thereon which, when executed by the processor, cause the computer to display, by way of the display device, a virtual target representing the target tissue.

9. The system according to claim 8, further configured to generate an overlay representation of a location within the luminal network of the patient where a biopsy has been taken; and
display the overlay representation on a corresponding portion of the virtual target.

10. The system according to claim 9, wherein the memory has further instructions stored thereon which, when executed by the processor, cause the computer to receive, by way of the input device, an input indicating that a current location of the distal portion of the catheter within the luminal network of the patient corresponds to the location where the biopsy has been taken within the luminal network of the patient; and
in response to the receiving of the input, identify a location within the virtual target representing the location where the biopsy has been taken within the luminal network of the patient; preferably further configured to indicate, by way of the display device, a location within the virtual target where a biopsy needs to be taken.

11. The system according to claim 10, wherein an attribute of the displayed virtual target changes based on changes in the location of the distal portion of the catheter within the luminal network of the patient.

12. The system according to any preceding claim, wherein the memory has further instructions stored thereon which, when executed by the processor, cause the computer to receive, by way of the input device, an input indicating the location of the target tissue on the ultrasound image displayed on the display device; and
to generate the location data corresponding to the location of the target tissue based on the received input indicating the location of the target tissue on the ultrasound image.

13. A non-transitory computer-readable medium storing instructions that, when executed by a processor, cause the processor to perform a method for facilitating electromagnetic navigation bronchoscopy using ultrasound, the method comprising:
(i) repeatedly, while a catheter and an ultrasound probe inserted therein are navigated throughout a luminal network of a patient, and until an instruction to store location data corresponding to a location of target tissue is received:
receiving, from an electromagnetic sensor disposed on a distal portion of the catheter, an electromagnetic sensor signal value corresponding to a location of the distal portion of the catheter within the luminal network of the patient;
storing in a memory the received electromagnetic sensor signal value;
displaying, on a display device, in a survey window of a user interface, a marker indicating the location of the distal portion of the catheter;
determining a location of a distal portion of the ultrasound probe based on the electromagnetic sensor signal value corresponding to the location of the distal portion of the catheter and a known distance by which the distal portion of the ultrasound probe protrudes from the distal portion of the catheter;
receiving ultrasound image data from the ultrasound probe, wherein the ultrasound probe protrudes from the distal portion of the catheter; and
displaying, by way of the display device, an ultrasound image based on the received ultrasound image data;
(ii) once an instruction to store location data corresponding to a location of target tissue, while at least a portion of the target tissue is shown in the displayed ultrasound image, is received by way of an input device, storing the location data corresponding to the location of the target tissue in the memory, wherein the location data corresponding to the location of the target tissue is based on the received electromagnetic sensor signal value corresponding to the location of the distal portion of the catheter, while the at least a portion of the target tissue is shown in the displayed ultrasound image.

## Patentansprüche

1. System zum Erleichtern einer elektromagnetischen Navigationsbronchoskopie unter Verwendung von Ultraschall, umfassend:
eine Ultraschallsonde;
einen Katheter, der konfiguriert ist, um die Ultraschallsonde aufzunehmen, wobei der Katheter einen distalen Abschnitt einschließt, auf dem ein elektromagnetischer Sensor angeordnet ist,
eine Anzeigevorrichtung,
eine Eingabevorrichtung, und
einen Computer, der einschließt:
einen Prozessor; und
einen Speicher, der mit dem Prozessor gekoppelt ist, wobei der Speicher Anweisungen aufweist, die darauf gespeichert sind, die, wenn sie durch den Prozessor ausgeführt werden, den Computer veranlassen:
(i) wiederholt, während der Katheter und die darin eingeführte Ultraschallsonde durch ein luminales Netzwerk eines Patienten bewegt werden, und bis eine Anweisung, Positionsdaten zu speichern, die einer Position von Zielgewebe entsprechen, empfangen wird:
Empfangen, von dem elektromagnetischen Sensor, eines Signalwerts des elektromagnetischen Sensors, der einer Position des distalen Abschnitts des Katheters innerhalb des luminalen Netzwerks eines Patienten entspricht,
Speichern in dem Speicher der Vielzahl von empfangenen Signalwerten des elektromagnetischen Sensors,
Anzeigen, auf der Anzeigevorrichtung in einem Übersichtsfenster einer Benutzerschnittstelle, einer Markierung, die die Position des distalen Abschnitts des Katheters angibt,
Bestimmen einer Position eines distalen Abschnitts der Ultraschallsonde basierend auf dem Signalwert des elektromagnetischen Sensors, der der Position des distalen Abschnitts des Katheters entspricht, und einem bekannten Abstand, den der distale Abschnitt der Ultraschallsonde aus dem distalen Abschnitt des Katheters hervorsteht;
Empfangen von Ultraschallbilddaten von der Ultraschallsonde, wobei die Ultraschallsonde aus dem distalen Abschnitt des Katheters hervorsteht; und
Anzeigen, über die Anzeigevorrichtung, eines Ultraschallbildes basierend auf den empfangenen Ultraschallbilddaten;
(ii) sobald eine Anweisung, Positionsdaten zu speichern, die einer Position von Zielgewebe entsprechen, während mindestens ein Abschnitt des Zielgewebes in dem angezeigten Ultraschallbild gezeigt wird, über die Eingabevorrichtung empfangen wird, Speichern der Positionsdaten, die der Position Zielgewebes entsprechen, in dem Speicher, wobei die Positionsdaten, die der Position des Zielgewebes entsprechen, auf dem empfangenen Signalwert des elektromagnetischen Sensors basieren, der der Position des distalen Abschnitts des Katheters entspricht, während der mindestens eine Abschnitt des Zielgewebes in dem angezeigten Ultraschallbild gezeigt wird.

2. System nach Anspruch 1, wobei der Speicher weitere Anweisungen aufweist, die darauf gespeichert sind, die, wenn sie durch den Prozessor ausgeführt werden, den Computer veranlassen, über die Anzeigevorrichtung, das Übersichtsfenster angrenzend an das Ultraschallbild anzuzeigen.

3. System nach Anspruch 1, wobei die Vielzahl von Signalwerten des elektromagnetischen Sensors zu einer Vielzahl von unterschiedlichen Zeiten empfangen werden.

4. System nach Anspruch 1, wobei einer der Vielzahl von Signalwerten des elektromagnetischen Sensors, die einer der Vielzahl von Positionen des distalen Abschnitts des Katheters entsprechen, gespeichert wird, wenn die eine der Vielzahl von Positionen über einen vorbestimmten Abstand von einer zuvor gespeicherten Position des distalen Abschnitts des Katheters verfügt.

5. System nach einem vorstehenden Ansprüche, wobei der Speicher weitere Anweisungen aufweist, die darauf gespeichert sind, die wenn sie durch den Prozessor ausgeführt werden, den Computer veranlassen, von einem zusätzlichen elektromagnetischen Sensor, der mit dem distalen Abschnitt der Ultraschallsonde gekoppelt ist, einen zusätzlichen Signalwert des elektromagnetischen Sensors zu empfangen, der einer Position des distalen Abschnitts der Ultraschallsonde innerhalb des luminalen Netzwerks des Patienten entspricht, wobei das Bestimmen der Position des distalen Abschnitts der Ultraschallsonde auf dem zusätzlichen Signalwert des elektromagnetischen Sensors basiert.

6. System nach einem der vorstehenden Ansprüche, wobei der Speicher weitere Anweisungen aufweist, die darauf gespeichert sind, die, wenn sie durch den Prozessor ausgeführt werden, den Computer veranlassen, die Position des Zielgewebes relativ zu der Position des distalen Abschnitts der Ultraschallsonde zu bestimmen; und
die Positionsdaten zu erzeugen, die der Position des Zielgewebes entsprechen, basierend auf der Position des Zielgewebes relativ zu der Position des distalen Abschnitts der Ultraschallsonde; und/oder den Computer ferner veranlassen, die Ultraschallbilddaten zu verarbeiten; und
die Position des Zielgewebes innerhalb des luminalen Netzwerks des Patienten basierend auf dem Verarbeiten der Ultraschallbilddaten und der Position des distalen Abschnitts der Ultraschallsonde zu bestimmen.

7. System nach einem vorstehenden Ansprüche, wobei der Speicher weitere Anweisungen aufweist, die darauf gespeichert sind, die, wenn sie durch den Prozessor ausgeführt werden, den Computer veranlassen die Positionsdaten, die der Position des Zielgewebes entsprechen, basierend auf dem Signalwert des elektromagnetischen Sensors, der der Position des distalen Abschnitts des Katheters entspricht, zu einer Zeit zu erzeugen, zu der die Anweisung, den Signalwert des elektromagnetischen Sensors, der einer Position des Zielgewebes entspricht, zu speichern, empfangen wird.

8. System nach einem der vorstehenden Ansprüche, wobei der Speicher weitere Anweisungen aufweist, die darauf gespeichert sind, die, wenn sie durch den Prozessor ausgeführt werden, den Computer veranlassen, über die Anzeigevorrichtung, ein virtuelles Ziel anzuzeigen, das das Zielgewebe darstellt.

9. System nach Anspruch 8, das ferner konfiguriert ist, um eine Überlagerungsdarstellung einer Position innerhalb des luminalen Netzwerks des Patienten zu erzeugen, wo eine Biopsie vorgenommen wurde; und
um die Überlagerungsdarstellung auf einem entsprechenden Abschnitt des virtuellen Ziels anzuzeigen.

10. System nach Anspruch 9, wobei der Speicher weitere Anweisungen aufweist, die darauf gespeichert sind, die, wenn sie durch den Prozessor ausgeführt werden, den Computer veranlassen, über die Eingabevorrichtung, eine Eingabe zu empfangen, die angibt, dass eine aktuelle Position des distalen Abschnitts des Katheters innerhalb des luminalen Netzwerks des Patienten der Position entspricht, wo die Biopsie innerhalb des luminalen Netzwerks des Patienten vorgenommen wurde; und
als Reaktion auf das Empfangen der Eingabe, eine Position innerhalb des virtuellen Ziels zu identifizieren, das die Position darstellt, wo die Biopsie innerhalb des luminalen Netzwerks des Patienten vorgenommen wurde; wobei es vorzugsweise ferner konfiguriert ist, um, über die Anzeigevorrichtung, eine Position innerhalb des virtuellen Ziels anzugeben, wo eine Biopsie vorgenommen werden muss.

11. System nach Anspruch 10, wobei sich eine Eigenschaft des angezeigten virtuellen Ziels basierend auf Änderungen in der Position des distalen Abschnitts des Katheters innerhalb des luminalen Netzwerks des Patienten ändert.

12. System nach einem der vorstehenden Ansprüche, wobei der Speicher weitere Anweisungen aufweist, die darauf gespeichert sind, die, wenn sie durch den Prozessor ausgeführt werden, den Computer veranlassen, über die Eingabevorrichtung, eine Eingabe zu empfangen, die die Position des Zielgewebes auf dem Ultraschallbild angibt, das auf der Anzeigevorrichtung angezeigt wird, und
die Positionsdaten zu erzeugen, die der Position des Zielgewebes entsprechen, basierend auf der empfangenen Eingabe, die die Position des Zielgewebes auf dem Ultraschallbild angibt.

13. Nicht flüchtiges computerlesbares Medium, das Anweisungen speichert, die, wenn sie durch einen Prozessor ausgeführt werden, den Prozessor veranlassen, ein Verfahren zum Erleichtern einer elektromagnetischen Navigationsbronchoskopie unter Verwendung von Ultraschall durchzuführen, das Verfahren umfassend:
(i) wiederholt, während ein Katheter und eine darin eingeführte Ultraschallsonde durch ein luminales Netzwerk eines Patienten bewegt werden, und bis eine Anweisung, Positionsdaten zu speichern, die einer Position von Zielgewebe entsprechen, empfangen wird:
Empfangen, von einem elektromagnetischen Sensor, der auf einem distalen Abschnitt des Katheters angeordnet ist, eines Signalwerts des elektromagnetischen Sensors, der einer Position des distalen Abschnitts des Katheters innerhalb des luminalen Netzwerks des Patienten entspricht,
Speichern in einem Speicher des empfangenen Signalwert des elektromagnetischen Sensors,
Anzeigen, auf einer Anzeigevorrichtung, in einem Übersichtsfenster einer Benutzerschnittstelle, einer Markierung, die die Position des distalen Abschnitts des Katheters angibt,
Bestimmen einer Position eines distalen Abschnitts der Ultraschallsonde basierend auf dem Signalwert des elektromagnetischen Sensors, der der Position des distalen Abschnitts des Katheters entspricht, und einem bekannten Abstand, den der distale Abschnitt der Ultraschallsonde aus dem distalen Abschnitt des Katheters hervorsteht;
Empfangen von Ultraschallbilddaten von der Ultraschallsonde, wobei die Ultraschallsonde aus dem distalen Abschnitt des Katheters hervorsteht; und
Anzeigen, über die Anzeigevorrichtung, eines Ultraschallbildes basierend auf den empfangenen Ultraschallbilddaten;
(ii) sobald eine Anweisung, Positionsdaten zu speichern, die einer Position von Zielgewebe entsprechen, während mindestens ein Abschnitt des Zielgewebes in dem angezeigten Ultraschallbild gezeigt wird, über eine Eingabevorrichtung empfangen wird, Speichern der Positionsdaten, die der Position Zielgewebes entsprechen, in dem Speicher, wobei die Positionsdaten, die der Position des Zielgewebes entsprechen, auf dem empfangenen Signalwert des elektromagnetischen Sensors basieren, der der Position des distalen Abschnitts des Katheters entspricht, während der mindestens eine Abschnitt des Zielgewebes in dem angezeigten Ultraschallbild gezeigt wird.

## Revendications

1. Système destiné à faciliter la bronchoscopie par navigation électromagnétique à l'aide d'ultrasons, comprenant :
une sonde à ultrasons ;
un cathéter conçu pour recevoir la sonde à ultrasons, le cathéter comportant une partie distale sur laquelle un capteur électromagnétique est disposé ;
un dispositif d'affichage ;
un dispositif d'entrée ; et
un ordinateur comportant :
un processeur ; et
une mémoire couplée au processeur, la mémoire ayant des instructions stockées sur celle-ci qui, lorsqu'elles sont exécutées par le processeur, amènent l'ordinateur à :
(i) de manière répétée, pendant que le cathéter et la sonde à ultrasons insérée dans celui-ci se déplacent dans le réseau luminal d'un patient, et jusqu'à ce qu'une instruction de stockage de données de localisation correspondant à l'emplacement d'un tissu cible soit reçue :
recevoir, à partir du capteur électromagnétique, une valeur de signal de capteur électromagnétique correspondant à un emplacement de la partie distale du cathéter à l'intérieur du réseau luminal d'un patient ;
stocker, dans la mémoire, la pluralité de valeurs de signal de capteur électromagnétique reçues ;
afficher, sur le dispositif d'affichage dans une fenêtre de relevé d'une interface utilisateur, un marqueur indiquant l'emplacement de la partie distale du cathéter ;
déterminer un emplacement d'une partie distale de la sonde à ultrasons en fonction de la valeur de signal de capteur électromagnétique correspondant à l'emplacement de la partie distale du cathéter et d'une distance connue à laquelle la partie distale de la sonde à ultrasons fait saillie par rapport à la partie distale du cathéter ;
recevoir des données d'image ultrasonore de la sonde à ultrasons, dans lequel la sonde à ultrasons fait saillie par rapport à la partie distale du cathéter ; et
afficher, au moyen du dispositif d'affichage, une image ultrasonore en fonction des données d'image ultrasonore reçues ;
(ii) une fois qu'une instruction pour stocker des données de localisation correspondant à un emplacement du tissu cible, pendant qu'au moins une partie du tissu cible est représentée dans l'image ultrasonore affichée, est reçue au moyen du dispositif d'entrée, stocker les données de localisation correspondant à l'emplacement du tissu cible dans la mémoire, dans lequel les données de localisation correspondant à l'emplacement du tissu cible sont basées sur la valeur de signal de capteur électromagnétique reçue correspondant à l'emplacement de la partie distale du cathéter, pendant que l'au moins une partie du tissu cible est représentée dans l'image ultrasonore affichée.

2. Système selon la revendication 1, dans lequel la mémoire a des instructions supplémentaires stockées sur celle-ci qui, lorsqu'elles sont exécutées par le processeur, amènent l'ordinateur à afficher, au moyen du dispositif d'affichage, la fenêtre de relevé adjacente à l'image ultrasonore.

3. Système selon la revendication 1, dans lequel la pluralité de valeurs de signaux de capteur électromagnétique sont reçues à une pluralité de moments distincts.

4. Système selon la revendication 1, dans lequel l'une parmi la pluralité de valeurs de signal de capteur électromagnétique correspondant à l'un parmi la pluralité d'emplacements de la partie distale du cathéter est stockée lorsque l'un parmi la pluralité d'emplacements est à une distance prédéterminée d'un emplacement précédemment stocké de la partie distale du cathéter.

5. Système selon l'une quelconque revendication précédente, dans lequel la mémoire a des instructions supplémentaires stockées sur celle-ci qui, lorsqu'elles sont exécutées par le processeur, amènent l'ordinateur à recevoir, à partir d'un capteur électromagnétique supplémentaire, couplé à la partie distale de la sonde à ultrasons, une valeur de signal de capteur électromagnétique supplémentaire correspondant à un emplacement, à l'intérieur du réseau luminal du patient, de la partie distale de la sonde à ultrasons, dans lequel la détermination de l'emplacement de la partie distale de la sonde à ultrasons est basée sur la valeur de signal de capteur électromagnétique supplémentaire.

6. Système selon l'une quelconque revendication précédente, dans lequel la mémoire a des instructions supplémentaires stockées sur celle-ci qui, lorsqu'elles sont exécutées par le processeur, amènent l'ordinateur à déterminer l'emplacement du tissu cible par rapport à l'emplacement de la partie distale de la sonde à ultrasons ; et
générer les données de localisation correspondant à l'emplacement du tissu cible en fonction de l'emplacement du tissu cible par rapport à l'emplacement de la partie distale de la sonde à ultrasons ; et/ou amènent l'ordinateur à traiter en outre les données d'image ultrasonore ; et
déterminer, en fonction du traitement des données d'image ultrasonore et de l'emplacement de la partie distale de la sonde à ultrasons, l'emplacement du tissu cible à l'intérieur du réseau luminal du patient.

7. Système selon l'une quelconque revendication précédente, dans lequel la mémoire a des instructions supplémentaires stockées sur celle-ci qui, lorsqu'elles sont exécutées par le processeur, amènent l'ordinateur à générer les données de localisation correspondant à l'emplacement du tissu cible en fonction de la valeur de signal de capteur électromagnétique correspondant à l'emplacement de la partie distale du cathéter à un moment où l'instruction de stocker la valeur de signal de capteur électromagnétique correspondant à un emplacement du tissu cible est reçue.

8. Système selon l'une quelconque revendication précédente, dans lequel la mémoire a des instructions supplémentaires stockées sur celle-ci qui, lorsqu'elles sont exécutées par le processeur, amènent l'ordinateur à afficher, au moyen du dispositif d'affichage, une cible virtuelle représentant le tissu cible.

9. Système selon la revendication 8, configuré en outre pour générer une représentation de superposition d'un emplacement à l'intérieur du réseau luminal du patient où une biopsie a été effectuée ; et
pour afficher la représentation de superposition sur une partie correspondante de la cible virtuelle.

10. Système selon la revendication 9, dans lequel la mémoire a des instructions supplémentaires stockées sur celle-ci qui, lorsqu'elles sont exécutées par le processeur, amènent l'ordinateur à recevoir, au moyen du dispositif d'entrée, une entrée indiquant qu'un emplacement actuel de la partie distale du cathéter à l'intérieur du réseau luminal du patient correspond à l'emplacement où la biopsie a été effectuée à l'intérieur du réseau luminal du patient ; et
en réponse à la réception de l'entrée, identifier un emplacement à l'intérieur de la cible virtuelle représentant l'emplacement où la biopsie a été effectuée à l'intérieur du réseau luminal du patient ; de préférence configuré en outre pour indiquer, au moyen du dispositif d'affichage, un emplacement à l'intérieur de la cible virtuelle où une biopsie doit être effectuée.

11. Système selon la revendication 10, dans lequel un attribut de la cible virtuelle affichée change en fonction de changements dans l'emplacement de la partie distale du cathéter à l'intérieur du réseau luminal du patient.

12. Système selon l'une quelconque revendication précédente, dans lequel la mémoire a des instructions supplémentaires stockées sur celle-ci qui, lorsqu'elles sont exécutées par le processeur, amènent l'ordinateur à recevoir, au moyen du dispositif d'entrée, une entrée indiquant l'emplacement du tissu cible sur l'image ultrasonore affichée sur le dispositif d'affichage ; et
générer les données de localisation correspondant à l'emplacement du tissu cible en fonction de l'entrée reçue indiquant l'emplacement du tissu cible sur l'image ultrasonore.

13. Support non transitoire lisible par ordinateur stockant des instructions qui, lorsqu'elles sont exécutées par un processeur, amènent le processeur à exécuter un procédé pour faciliter une bronchoscopie par navigation électromagnétique à l'aide d'ultrasons, le procédé comprenant :
(i) de manière répétée, pendant qu'un cathéter et une sonde à ultrasons insérée dans celui-ci se déplacent dans un réseau luminal d'un patient, et jusqu'à ce qu'une instruction de stockage de données de localisation correspondant à un emplacement d'un tissu cible soit reçue :
la réception, à partir d'un capteur électromagnétique disposé sur une partie distale du cathéter, d'une valeur de signal de capteur électromagnétique correspondant à un emplacement de la partie distale du cathéter à l'intérieur du réseau luminal du patient ;
le stockage, dans une mémoire, de la valeur de signal de capteur électromagnétique reçue ;
l'affichage, sur un dispositif d'affichage, dans une fenêtre de relevé d'une interface utilisateur, d'un marqueur indiquant l'emplacement de la partie distale du cathéter ;
la détermination d'un emplacement d'une partie distale de la sonde à ultrasons en fonction de la valeur de signal de capteur électromagnétique correspondant à l'emplacement de la partie distale du cathéter et d'une distance connue à laquelle la partie distale de la sonde à ultrasons fait saillie par rapport à la partie distale du cathéter ;
la réception de données d'image ultrasonore de la sonde à ultrasons, dans lequel la sonde à ultrasons fait saillie par rapport à la partie distale du cathéter ; et
l'affichage, au moyen du dispositif d'affichage, d'une image ultrasonore en fonction des données d'image ultrasonore reçues ;
(ii) une fois qu'une instruction pour stocker des données de localisation correspondant à un emplacement de tissu cible, pendant qu'au moins une partie du tissu cible est représentée dans l'image ultrasonore affichée, est reçue au moyen d'un dispositif d'entrée, le stockage des données de localisation correspondant à l'emplacement du tissu cible dans la mémoire, dans lequel les données de localisation correspondant à l'emplacement du tissu cible sont basées sur la valeur de signal de capteur électromagnétique reçue correspondant à l'emplacement de la partie distale du cathéter, pendant que l'au moins une partie du tissu cible est représentée dans l'image ultrasonore affichée.
